# EUROPÄISCHE PATENTSCHRIFT

(11) **EP 1 093 774 B1**
(45) Veröffentlichungstag und Bekanntmachung des Hinweises auf die Patenterteilung: **19.06.2002**
(21) Anmeldenummer: 99121106.1
(22) Anmeldetag: 21.10.1999
(51) Int. Cl.: A61F 2/08

(54) **Interferenzschraube**
Interference screw
Vis d'interférence

(43) Veröffentlichungstag der Anmeldung: 25.04.2001
(73) Patentinhaber: Karl Storz GmbH & Co. KG, 78532 Tuttlingen (DE)
(72) Erfinder: Strobel, Michael, PD Dr.med., 94315 Straubing (DE); Weiler, Andreas, Dr., 10550 Berlin (DE); Timmermans, André, 7261 HJ Ruurlo (NL)
(74) Vertreter: Weller, Wolfgang, Dr.

(56) Entgegenhaltungen:
- EP-A- 0 669 110
- DE-U- 29 621 340
- FR-A- 2 717 070
- FR-A- 2 745 999

## Beschreibung

Die Erfindung betrifft eine Interferenzschraube zum Verankern eines Sehnentransplantates in einer Öffnung in einem Knochen, mit einem Schraubenkörper, der ein Kopfende und ein Eintreibende aufweist, und der mit einem Außengewinde versehen ist, dessen Kontur sich vom Eintreibende zum Kopfende hin verändert.

Eine derartige Interferenzschraube ist aus der EP-A-0 669 110 bekannt.

Interferenzschrauben sind allgemein aus OP-JOURNAL 14 (1998) S. 278-284 "Biodegradierbare Interferenzschrauben in der Kreuzbandchirurgie" A. Weiler et al., Georg Thieme Verlag Stuttgart, New York bekannt.

Interferenzschrauben dienen dazu, ein Sehnentransplantat oder ein Sehnenimplantat in einem Knochen zu verankern. Dazu wird in den Knochen ein Kanal gebohrt, in den das Sehnentransplantat eingezogen wird. Die Interferenzschraube ist nun dazu vorgesehen, in einen Zwischenraum zwischen Sehnentransplantat und der Innenwand des Bohrkanales eingedreht zu werden, so daß dann das Sehnentransplantat zwischen der Schraube und der Innenwand des Bohrkanales geklemmt ist. Da auf eine solche Sehne, beispielsweise bei einem Kreuzbandersatz in einem Kniegelenk, erhebliche Kräfte einwirken, muß die Klemmkraft entsprechend groß sein, um eine dauerhafte Verankerung sicherzustellen. Dazu ist die Interferenzschraube mit einem Außengewinde versehen, das sich in das Knochenmaterial in der Innenseite des Bohrkanales hineinfrißt. Gleichzeitig tritt aber das Außengewinde auch in Eingriff mit dem zu verankernden Sehnentransplantat.

Aus der US-A-5 383 878 ist bekannt, das Außengewinde als stumpfes Rundgewinde auszubilden, das, längs der Schraubenlängsachse gesehen, eine etwa sinusförmig gewellte Kontur aufweist.

Interferenzschrauben mit einem stumpfen Gewinde lassen sich schwer eindrehen, was bei der klinischen Anwendung von großem Nachteil ist.

Aus der US-A-5 688 285 ist eine Interferenzschraube bekannt, deren Außengewinde mit einem scharfen Spitzgewinde versehen ist. Ein scharfes Gewinde kann beim Eindrehen das Sehnentransplantat auftrennen oder soweit schwächen, daß es bei Belastungen von der Verankerungsstelle abreißt. Daher haben solche Interferenzschrauben hauptsächlich bei der sogenannten BTB-Technik (Bone-Tendon-Bone) Einsatz gefunden, d.h. das Sehnentransplantat ist in dem Bereich, in dem es mit dem scharfen Außengewinde der Interferenzschraube in Eingriff kommt, mit einem Knochenstück ummantelt, so daß sich dann letztendlich die Interferenzschraube beim Verankern in das das Sehnenende umgebende Knochenmaterial einerseits und das Knochenmaterial der Innenwand des Bohrkanals andererseits einfrißt.

Aus der FR-A-2 717 070 ist eine Interferenzschraube bekannt, deren Außengewinde als stumpfes Rundgewinde ausgebildet ist. Im Bereich des Eintreibendes verjüngt sich die Außenkontur nach und nach, wozu von den runden Spitzen Material abgetragen ist, diese also gekappt sind. Dadurch entstehen mehr oder weniger eckige trapezgewindeartige Abschnitte im konisch verjüngten Bereich.

Aus der eingangs genannten EP-A-0 669 110 ist eine Interferenzschraube kannt, die ein Außengewinde in Form eines Trapezgewindes aufweist. Auch hier verjüngt sich der Körper in Richtung Eintrittsende konisch. Die Kontur verändert sich vom Eintreibende zum Kopfende dahingehend, daß die Breite der äußeren Fläche des Trapezgewindes immer größer wird.

Aus der FR-A-2 745 999 ist eine Interferenzschraube mit einem stumpfen Rundgewinde bekannt, deren Spitze sich stark konisch verjüngt. In diesem stark konisch verjüngten Bereich sind die sanften Rundungen des Rundgewindes entsprechend dem Konuswinkel abgekappt, so daß einem Trapezgewinde ähnliche Stümpfe verbleiben.

Es ist Aufgabe der vorliegenden Erfindung, eine Interferenzschraube zu schaffen, die ein einfaches und sicheres Verankern eines Sehnentransplantates möglich macht.

Erfindungsgemäß wird die Aufgabe dadurch gelöst, daß das Außengewinde im Bereich des Eintreibendes als scharfes Gewinde ausgebildet ist und in einem nachlaufenden Abschnitt als stumpfes Gewinde ausgebildet ist, wobei die scharfen Gewindebereiche bis in etwa zum maximalen Kerndurchmesser reichen, so daß diese Einlaufwege für die nachlaufenden stumpfen Abschnitte in der Öffnung des Knochens einschneiden.

Beim Eindrehen der Interferenzschraube schaffen die scharfen Gewindebereiche am Eintreibende klar definierte Einlaufwege für die nachfolgenden stumpfen Abschnitte des Gewindes. Es werden etwa komplementäre schraubenlinienförmige Linien an der Innenseite des Bohrkanales eingeschnitten. In diese Einschnitte können dann die stumpfen Bereiche des Gewindes einfach eingedreht werden bzw. einlaufen, wobei dann diese stumpfen Gewindebereiche für die ausreichende Kompression sorgen, um das Sehnentransplantat an der Innenseite des Bohrkanales zu verankern. Dadurch ist die Interferenzschraube einfach und gut geführt einzudrehen. Als weiterer Vorteil ist sichergestellt, daß im Bereich der stumpfen Gewindeabschnitte das Transplantatgewebe nicht beeinträchtigt, d.h. nicht ein- oder angeschnitten wird.

In einer weiteren Ausgestaltung der Erfindung ist zumindest ein Gewindeumlauf scharf ausgebildet.

Durch die Ausbildung zumindest eines Umlaufes des Gewindes als scharfes schneidendes Gewinde ist sichergestellt, daß eine um den gesamten Umfang der Kanalwand eingeschnittene Führungslinie oder Führungsnut vorhanden ist, in die dann die nachlaufenden stumpfen Bereiche des Gewindes exakt einlaufen können.

In einer weiteren Ausgestaltung der Erfindung ist der Schraubenkörper am Eintreibende verjüngt, und das scharfe Gewinde reicht soweit, bis etwa der maximale Außendurchmesser des Außengewindes erreicht ist.

Diese Maßnahme hat den Vorteil, daß durch das verjüngte Ende die Interferenzschraube in den Freiraum zwischen Sehnenersatz und Bohrkanal angesetzt und dann korrekt ausgerichtet eingedreht werden kann. Da das Material des Sehnentransplantates eine gewisse Kompressibilität aufweist, kann das Material im Bereich der Gewindegänge am Eintreibende seitlich ausweichen, so daß die Gefahr von Beeinträchtigungen durch das Gewinde weiter herabgesetzt ist. Dadurch sind dann auch Korrekturen durch ein Wiederausdrehen einer einmal angesetzten Schraube nicht mehr notwendig. Dennoch ist sichergestellt, daß die bis in den maximalen Kerndurchmesser reichenden scharfen Gewindeabschnitte die Einlaufkontur in die Innenwand des Knochenkanales einschneiden. Dadurch ist die Handhabung weiter erleichtert.

In einer weiteren Ausgestaltung der Erfindung ist der Übergang vom scharfen zum stumpfen Gewinde fließend.

Diese Maßnahme hat den Vorteil, daß das Einführen der nachlaufenden stumpfen Gewindebereiche in die von den scharfen Gewindebereichen vorgeschnittenen schraubenlinienförmigen Nuten an der Innenwand des Bohrkanales äußerst sanft abläuft.

In einer weiteren Ausgestaltung der Erfindung ist das Außengewinde als Sägengewinde ausgebildet.

Diese Maßnahme hat den Vorteil, daß die vorlaufende Flanke des Sägezahnprofiles sich relativ sanft in die Materialien einfressen kann. Bei dem relativ kompressiblen Sehnentransplantatmaterial wird dieses entsprechend radial verschoben oder verdichtet, ohne daß dadurch das Transplantat nachteilig beeinträchtigt wird. Sowohl das Knochenmaterial als auch das Sehnenmaterial hat ein gewisses Rückstellvermögen, so daß dann dieses Material im Bereich der nachlaufenden Flanke wieder einrücken und einen mechanisch festen, sperrenden Verbund mit der Interferenzschraube bildet.

In einer weiteren Ausgestaltung der Erfindung ist die Interferenzschraube aus biodegradierbarem Material hergestellt. Diese Maßnahme hat den Vorteil, daß Knochenmaterial in den Raum nach und nach einwachsen kann, der von dem Material der Interferenzschraube belegt war. Dies stellt auf Dauer einen fest haftenden Sitz des Sehnentransplantates dadurch sicher, daß organische Verwachsungen mit dem Sehnentransplantat stattfinden, so daß eine Lockerung zwischen Interferenzschraube und verankertem Sehnentransplantat aufgrund von Belastungen oder degenerativen Verformungen ausgeschlossen sind, somit auf Dauer ein sicherer Sitz gewährleistet ist. Dieser eröffnet auch die Möglichkeit, bei Revisionen erneut Bohrungen anzubringen, um ein weiteres oder anderes Sehnentransplantat im selben Knochenbereich zu verankern, da durch das Nachwachsen wieder Knochenmaterial vorhanden ist, daß erneut angebohrt werden kann.

In einer weiteren Ausgestaltung der Erfindung ist der Schraubenkörper als Hohlkörper ausgebildet und in einer weiteren Ausgestaltung ist dieser mit mehreren Perforationen versehen.

Diese Maßnahmen haben den Vorteil, daß durch die Perforationen das Ausbilden eines dreidimensionalen Knochengerüstes im Bereich der Interferenzschraube weiter begünstigt ist. Über die Perforationen kann Knochenmaterial in den Innenraum des Hohlkörpers hineinwachsen oder mit gegebenenfalls in dem Innenraum des Hohlkörpers bereits vorhandenem Knochenmaterial, sei es als Knochenaufschlemmung oder Knochenschnipsel, verwachsen und eine innige Verbindung eintreten. Dieser Vorgang kann parallel zu dem biologischen Abbau des Materials des Schraubenkörpers verlaufen, so daß andauernd ein sicherer Sitz des Transplantates gewährleistet ist und nach und nach mehr und mehr natürliches Knochenmaterial im Bereich dieser Transplantatstelle gebildet wird. Die Form, die Verteilung und die Anzahl der Perforationen ist so gewählt, daß eine ausreichende Stabilität des Hohlkörpers erhalten bleibt, dennoch zahlreiche Durchwachsstellen vorhanden sind.

In einer weiteren Ausgestaltung der Erfindung sind die Perforationen zwischen den Gewindegängen vorgesehen.

Diese Maßnahme hat den Vorteil, daß die Gewindegänge in einer durchgehend verlaufenden Schraubenlinienkontur gehalten werden können, was ein sanftes Eindrehen der Interferenzschraube erleichtert, die Perforationen zum Einwachsen des Knochengewebes dann in den Zwischenräumen im eigentlichen Schraubenkörper vorhanden sind.

Es versteht sich, daß die vorstehend genannten und die nachstehend noch zu erläuternden Maßnahmen nicht nur in den angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert.

Es zeigen:
- Fig. 1: eine Seitenansicht einer erfindungsgemäßen Interferenzschraube, wobei die rechte Hälfte als Längsschnitt dargestellt ist,
- Fig. 2: eine teilweise geschnittene Ansicht eines weiteren Ausführungsbeispiels einer Interferenzschraube, die zusätzlich mit Perforationen versehen und, wobei dargestellt ist, wie die Interferenzschraube gerade zum Verankern eines Transplantates in einen Bohrkanal eines Knochens mit Hilfe eines Werkzeuges eingetrieben wird, und
- Fig. 3: eine der Fig. 2 vergleichbare Darstellung nach vollständigem Eindrehen der Interferenzschraube mit verankertem Transplantat.

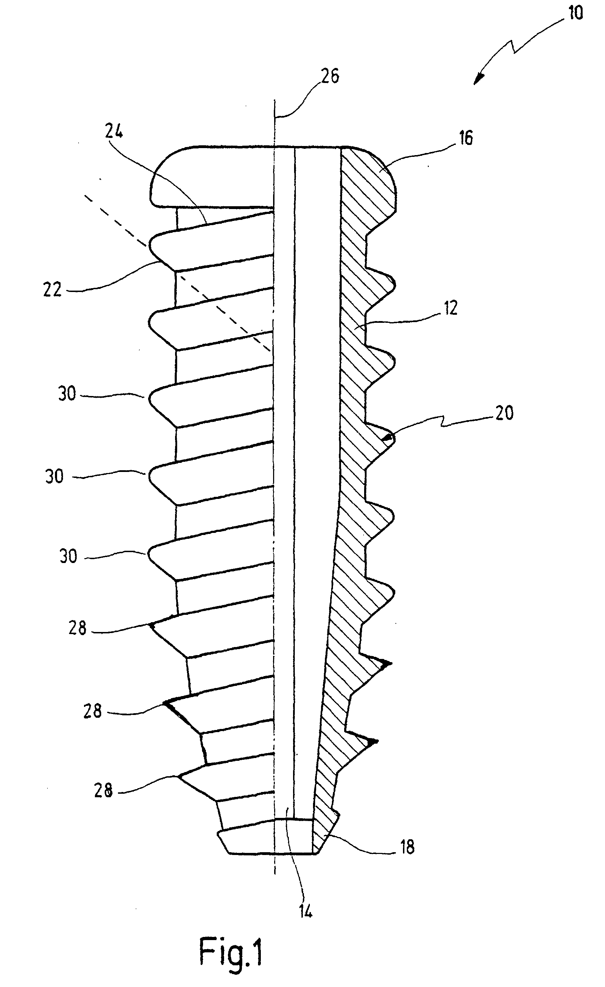

Eine in Fig. 1 dargestellte Interferenzschraube ist in ihrer Gesamtheit mit der Bezugsziffer 10 bezeichnet.

Die Interferenzschraube 10 weist einen hohlen Schraubenkörper 12 auf, der ein Kopfende 16 und ein Eintreibende 18 aufweist. Der etwa hohlzylindrische Schraubenkörper 12 verjüngt sich im Bereich des Eintreibendes 18.

Durch den Schraubenkörper 12 reicht vom Kopfende 16 bis zum Eintreibende 18 ein durchgehender Kanal 14, dessen Querschnitt, zumindest im Bereich des Kopfendes 16, sechseckförmig konturiert ist.

An seiner Außenseite ist der Schraubenkörper 12 mit einem Außengewinde 20 versehen, das vom Eintreibende 18 bis zum Kopfende 16 reicht.

Das Außengewinde 20 ist im dargestellten Ausführungsbeispiel als Sägengewinde profiliert. Die jeweils vorlaufende bzw. dem Eintreibende 18 zugewandte Flanke 22 steht zur Mittellängsachse 26 der Interferenzschraube 10 unter einem Winkel von etwa 45 Grad, wie dies in Fig. 1 auf der linken Hälfte in einer gestrichelten Linie dargestellt ist. Die andere Flanke 24 verläuft, vom Kopfende 16 zum Eintreibende 18 gesehen, um etwa 15 Grad nach unten geneigt, gegenüber einer senkrecht zur Achse 26 verlaufenden Ebene. Im Bereich des Eintreibendes 18 bis in etwa zum maximalen Kerndurchmessers des Schraubenkörpers 12 gehen die Flanken 22 bzw. 24 über eine scharfe Spitze 28 ineinander über.

In den nachlaufenden Abschnitten gehen die Flanken 22 und 24 jeweils über eine stumpfe Spitze 30 ineinander über.

Daraus resultiert ein erster Bereich des Außengewindes 20 in der Nähe des Eintreibendes 18 mit scharfen Gewinde, an den sich ein Bereich, der sich bis zum Kopfende 16 erstreckt, mit stumpfen Gewinde anschließt.

Die in Fig. 1 dargestellte Interferenzschraube 10 besteht aus einem biokompatiblen metallischen Material, nämlich aus Titan.

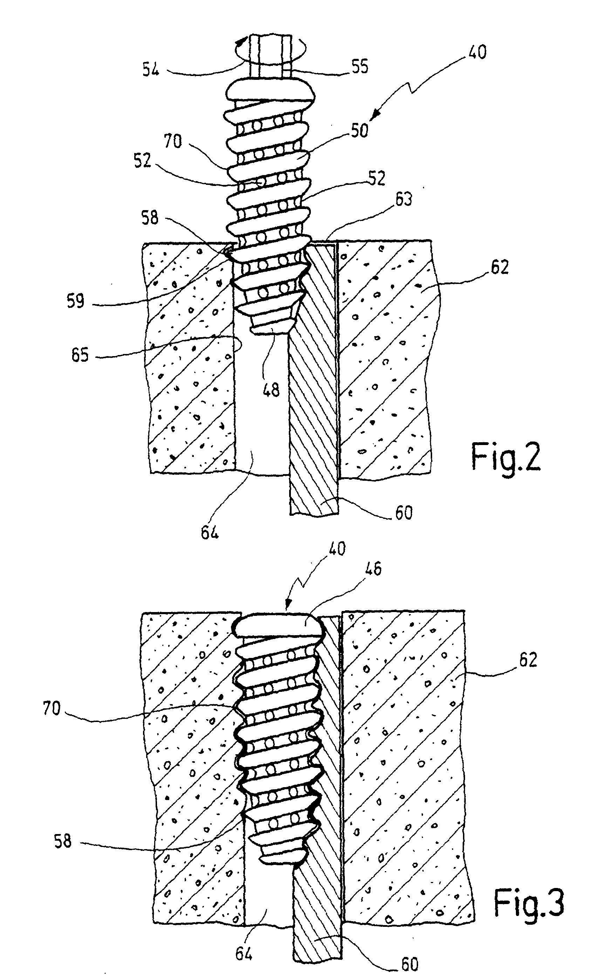

In den Fig. 2 und 3 ist ein weiteres Ausführungsbeispiel einer Interferenzschraube 40 dargestellt, die, was die Ausgestaltung deren Außengewinde 50 anbetrifft, identisch ausgebildet ist wie die in Fig. 1 beschriebene Interferenzschraube 10. Die Interferenzschraube 40 weist demzufolge ebenfalls ein Kopfende 46 und ein Eintreibende 48 auf.

Im Gegensatz zu dem in Fig. 1 dargestellten Ausführungsbeispiel besteht die Interferenzschraube 40 aus einem biodegradierbaren Material und ist außerdem, zwischen den Gewindegängen des Außengewindes 50 mit zahlreichen Perforationen 52 versehen. Beispiele für biodegradierbare Materialien sind: Polycaprolactone, Poly(L-Laktide), Polyglykolide, Poly(D,L-Laktide), Poly(D,L-Laktid-co-Glykolide), Poly(D,L-Laktid-co-Caprolactone), Polydioxanone, Copolyoxalate, Polycarbonate, z.B. Polyglykolid-co-Trimethylencarbonat, Poly(Glutamin-co-Leucine).

Die Perforationen 52 im dargestellten Ausführungsbeispiel bestehen in Form von kreisrunden Öffnungen, so daß über diese Öffnung eine Verbindung von der Außenseite in den Innenraum der ebenfalls als Hohlkörper ausgebildeten Interferenzschraube 40 vorhanden ist.

Der Sinn und Zweck dieser Ausgestaltung soll in Zusammenhang mit der Handhabung näher beschrieben werden, wobei die Handhabung der Interferenzschraube 40 zum Verankern eines Sehnentransplantates 60 identisch ist wie die Handhabung des in Fig. 1 dargestellten Ausführungsbeispiels einer Interferenzschraube 10 aus Metall. Dies beruht auf der Erkenntnis, daß biodegradierbare Interferenzschrauben über eine gleichhohe initiale Verankerungsfestigkeit wie Metallschrauben verfügen.

Der Vorgang einer Interferenzschraubenverankerung läuft wie folgt ab.

In dem Knochen 62, in den ein Sehnentransplantat 60 verankert werden soll, wird eine Öffnung 63 in Form eines Bohrkanales 64 bewerkstelligt. Der Durchmesser des Bohrkanales 64 wird so gewählt, daß in diesen das Sehnentransplantat 60 bzw. ein Ende davon eingeschoben werden kann.

Beim Ersatz eines Kreuzbandes werden entsprechende Bohrkanale 64 sowohl im Femur als auch in der Tibia bewerkstelligt und dort jeweils das Sehnentransplantat verankert, wie das Beispiel in Abb. 1 des vorgenannten Artikels im OP-JOURNAL 14 (1998) a.a.O. gezeigt ist.

Der Einfachheit halber ist in Fig. 2 nur dargestellt, wie ein Ende des Sehnentransplantates 60 verankert wird, das entsprechend andere wird dann gleichermaßen verankert.

Nach Einziehen des Sehnentransplantates 60 in den Bohrkanal 64 wird die Interferenzschraube 40 so angesetzt, daß sich deren Eintreibende 48 zwischen die Innenwand 65 des Bohrkanales 64 und das äußere Ende des Sehnentransplantates 60 schiebt. Dieses Einsetzen und Einschieben wird durch die Verjüngung im Bereich des Eintreibendes 48 begünstigt.

In das Kopfende 64 ist ein Werkzeug 54 eingeschoben, dessen Außenkontur der Querschnittskontur des inneren Kanales entspricht, beispielsweise einer sechseckförmigen Querschnittskontur. Durch Drehen des Werkzeuges 54, wie das durch einen Pfeil 55 angedeutet ist, kann dann die Interferenzschraube 40 eingedreht werden.

In Fig. 2 ist ein Situation dargestellt, bei der die scharfen Spitzen 58 des Außengewindes 50 im Bereich des Eintreibendes 28 gerade mit der Innenwand 65 des Bohrkanales 64, also mit dem Knochenmaterial, in Eingriff treten. Diese scharfen Spitzen 58 schneiden beim Eindrehen der Interferenzschraube 40 eine relativ schmale scharfkonturierte schraubenförmigen Linie in die Innenseite bzw. die Innenwand 65 des Bohrkanales 64 ein. An den Stellen, an denen das Außengewinde 50 mit den scharfen Spitzen 58 versehen ist, also etwa im Bereich der ersten drei Gewindeumläufe, schieben sich die vorlaufenden Flanken des Außengewindes 50 in das Material des Sehnentransplantates 60 hinein, ohne dieses zu verletzen, was durch das verjüngte Ende am Eintreibende 48 noch begünstigt wird.

Beim weiteren Eindrehen der Interferenzschraube 40 laufen dann die nachfolgenden stumpfen Gewindeabschnitte in das von den scharfen Spitzen 58 vorgeschnittene Innengewinde 59 in der Innenwand 65 sanft ein, erweitern diese und sorgen für die eigentliche radiale Kompression. Dabei graben sich die stumpfen Gewindeabschnitte, wie das in Fig. 3 auf der rechten Seite ersichtlich ist, entsprechend tief und fest in das Material des Sehnentransplantates 60 hinein. Da der Eingriff des Außengewindes 50 mit dem Material des Sehnentransplantates 60 mit hoher Kompression im wesentlichen im Bereich des stumpfen Gewindeabschnittes erfolgt, bestehen keine Beeinträchtigungen des Materials des Sehnentransplantates 16 durch das Außengewinde 50.

Die Interferenzschraube 40 ist einfach einzudrehen, sorgt für den ausreichenden Anpreßdruck, um eine sichere Verankerung des Sehnentransplantates 60 darzustellen.

Durch die Perforationen 52 kann nun Knochenmaterial in den Innenraum der Interferenzschraube 40 nach und nach hineinwachsen.

Der Innenraum kann mit Knochenmaterial, z.B. Knochenschlamm oder Knochenteilen, die beispielsweise aus der Beckenkammregion entnommen wurden, aufgefüllt sein. Dadurch bildet sich alsbald, z.B. nach sechs bis acht Wochen, ein fester Verbund aus Knochenmaterial mit dem Innenraum der Interferenzschraube.

Dadurch, daß das Material der Interferenzschraube biodegradierbar ist, wird dieses nach und nach abgebaut, verschwindet also nach und nach, so daß dann in die dadurch entstandenen Hohlräume weiteres Knochenmaterial nachwachsen kann. Durch diese vorteilhafte Ausgestaltung muß der zeitliche Ablauf der Biodegradation nicht mit der Knochenwachstumsgeschwindigkeit korreliert sein, dieses insbesondere viel rascher verlaufen kann, da dem Knochenmaterial über die Perforationen 52 ausreichend Platz zur Verfügung steht, schon vor oder während des Abbaus eine dreidimensional verästelte Knochenstruktur auszubilden. Hierdurch ergeben sich insbesondere in bezug auf Revisionseingriffe deutlich verbesserte Möglichkeiten.

## Patentansprüche

1. Interferenzschraube zum Verankern eines Sehnentransplantates (16) in einer Öffnung (63) in einem Knochen (62), mit einem Schraubenkörper (12), der ein Kopfende (16, 46) und ein Eintreibende (18, 48) aufweist, und der mit einem Außengewinde (20, 50) versehen ist, dessen Kontur sich vom Eintreibende (18, 48) zum Kopfende (16, 46) hin verändert, **dadurch gekennzeichnet, daß** das Außengewinde (20, 50) im Bereich des Eintreibendes (18, 48) als scharfes Gewinde ausgebildet ist und in einem nachlaufenden Abschnitt als stumpfes Gewinde ausgebildet ist, wobei die scharfen Gewindebereiche bis in etwa zum maximalen Kerndurchmesser reichen, so daß diese Einlaufwege für die nachlaufenden stumpfen Abschnitte in der Öffnung (68) des Knochens (62) einschneiden.

2. Interferenzschraube nach Anspruch 1, **dadurch gekennzeichnet, daß** zumindest ein Gewindeumlauf scharf ausgebildet ist.

3. Interferenzschraube nach Anspruch 1 oder 2, **dadurch gekennzeichnet, daß** der Schraubenkörper (12) am Eintreibende (18, 28) verjüngt ist, und das scharfe Gewinde soweit reicht, bis etwa der maximale Außendurchmesser des Außengewindes (20, 50) erreicht ist.

4. Interferenzschraube nach einem der Ansprüche 1 bis 3, **dadurch gekennzeichnet, daß** der Übergang vom scharfen zum stumpfen Gewinde fließend ist.

5. Interferenzschraube nach einem der Ansprüche 1 bis 4, **dadurch gekennzeichnet, daß** das Außengewinde (20, 50) als Sägengewinde ausgebildet ist.

6. Interferenzschraube nach einem der Ansprüche 1 bis 5, **dadurch gekennzeichnet, daß** diese aus biodegradierbarem Material hergestellt ist.

7. Interferenzschraube nach einem der Ansprüche 1 bis 6, **dadurch gekennzeichnet, daß** der Schraubenkörper als Hohlkörper ausgebildet ist.

8. Interferenzschraube nach einem der Ansprüche 1 bis 7, **dadurch gekennzeichnet, daß** der Schraubenkörper mit mehreren Perforationen (52) versehen ist.

9. Interferenzschraube nach Anspruch 8, **dadurch gekennzeichnet, daß** die Perforationen (52) zwischen den Gängen des Außengewindes (50) vorgesehen sind.

## Claims

1. Interference screw for anchoring a ligament transplant (16) in an opening (63) within a bone (62), comprising a screw body (12) having a head end (16, 46), a penetrating end (18, 48) and an outer threading (20, 50), a contour of said outer threading (20, 50) changes from the penetrating end (18, 48) to the head end (16, 46),
**characterized in that** the outer threading (20, 50) is formed as a sharp threading in the region of the penetrating end (18, 48) and is formed as a blunt threading in the following region, the regions of sharp threadings extend up to about the maximum core diameter, thereby cutting entrance paths for the following blunt regions into the opening (63) within the bone (62).

2. Interference screw of claim 1, **characterized in that** at least one thread winding is formed to be sharp.

3. Interference screw of claim 1 or 2, **characterized in that** the screw body (12) is tapered at the penetrating end (18, 28) and the sharp threading extends up to about where the maximum outer diameter of the outer threading (20, 50) is reached.

4. Interference screw of one of the claims 1 to 3, **characterized in that** the transition from the sharp to the blunt threading is smooth.

5. Interference screw of one of the claims 1 to 4, **characterized in that** the outer threading (20, 50) is formed as a buttress threading.

6. Interference screw of one of the claims 1 to 5, **characterized in that** it is made of biodegradable material.

7. Interference screw of one of the claims 1 to 6, **characterized in that** the screw body is formed as a hollow body.

8. Interference screw of one of the claims 1 to 7, **characterized in that** the screw body is provided with several perforations (52).

9. Interference screw of claim 8, **characterized in that** the perforations (52) are provided between the windings of the outer threading (50).

## Revendications

1. Vis d'interférence pour ancrer un transplant de tendon (16) dans une ouverture (63) d'un os (62), avec un corps de vis (12) qui comporte une extrémité de tête (46) et une extrémité d'introduction (18, 48), et qui est pourvue d'un filetage extérieur (20, 50) dont le contour varie depuis l'extrémité d'introduction (18, 48) et l'extrémité de tête (16, 46), **caractérisée en ce que** le filetage extérieur (20, 50) est conformé en filetage tranchant dans la zone d'extrémité d'introduction (18, 48) et en filetage émoussé dans une portion suivante, les zones tranchantes du filetage s'étendant approximativement jusqu'au diamètre maximal du noyau, ce qui fait que celles-ci entaillent, dans l'ouverture (68) de l'os (62), des voies d'introduction pour les portions émoussées suivantes.

2. Vis d'interférence selon la revendication 1, **caractérisée en ce qu'**au moins un tour de filetage est tranchant.

3. Vis d'interférence selon la revendication 1 ou 2, **caractérisée en ce que** le corps de vis (12) se rétrécit à l'extrémité d'introduction (18, 28), et le filetage tranchant s'étend jusqu'à ce que soit atteint approximativement le diamètre extérieur maximal du filetage extérieur (20, 50).

4. Vis d'interférence selon l'une des revendications 1 à 3, **caractérisée en ce que** la transition entre le filetage tranchant et le filetage obtus est continue.

5. Vis d'interférence selon l'une des revendications 1 à 4, **caractérisée en ce que** le filetage extérieur (20, 50) est conformé en filetage de scie.

6. Vis d'interférence selon l'une des revendications 1 à 5, **caractérisée en ce qu'**elle est fabriquée dans une matière biodégradable.

7. Vis d'interférence selon l'une des revendications 1 à 6, **caractérisée en ce que** le corps de vis est conformé en corps creux.

8. Vis d'interférence selon l'une des revendications 1 à 7, **caractérisée en ce que** le corps de vis est pourvu de plusieurs perforations (52).

9. Vis d'interférence selon la revendication 8, **caractérisée en ce que** les perforations (52) sont prévues entre les filets du filetage extérieur (50).
